(19) Europäisches Patentamt
European Patent Office
Office européen des brevets

(11) **EP 4 649 832 A1**

(12) **EUROPEAN PATENT APPLICATION**

(43) Date of publication:
**19.11.2025 Bulletin 2025/47**

(21) Application number: **24305771.8**

(22) Date of filing: **17.05.2024**

(51) International Patent Classification (IPC):
**A23J 1/18** (2006.01)    **A23J 3/20** (2006.01)
**A23L 31/15** (2016.01)    **A23L 33/145** (2016.01)
**A23L 33/195** (2016.01)    **B01D 61/14** (2006.01)
**B01D 61/58** (2006.01)    **C07K 1/34** (2006.01)
**C12N 1/06** (2006.01)    **C12N 1/08** (2006.01)

(52) Cooperative Patent Classification (CPC):
**A23J 1/18; A23J 3/20; A23L 31/15; A23L 33/145;
A23L 33/195; B01D 61/145; B01D 61/147;
C07K 1/34; C12N 1/063; C12N 1/08;
B01D 2311/2692**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(71) Applicant: **ROQUETTE FRERES**
**62136 Lestrem (FR)**

(72) Inventor: **The designation of the inventor has not
yet been filed**

(74) Representative: **Plasseraud IP**
**104 Rue de Richelieu
CS92104
75080 Paris Cedex 02 (FR)**

(54) **MICROORGANISM PROTEIN ISOLATE**

(57)    The present application relates to protein derived from microorganisms, and more specifically derived from yeasts, that possess low RNA content and high gelling capacity. This application also relates to an industrial process and industrial uses of such single cell proteins for human and animal nutrition, health and wellbeing.

**EP 4 649 832 A1**

## Description

The present disclosure relates to the field of proteins derived from microorganisms, and more specifically derived from yeasts, that can be widely used for human and animal nutrition, health and well-being.

## Background art

Along with carbohydrates and lipids, proteins make up a significant part of our diet. The required amount of protein is generally said to be between 12% and 20% of our daily food intake.

The proteins consumed are generally either of animal origin, such as meats, fish, eggs and milk products, or of plant origin, including cereals, oleaginous plants and leguminous plants.

In industrialized countries, protein intake comes predominantly from proteins of animal origin. It is important to note that many studies show that excessive consumption of proteins that are of animal origin, and significantly less plant proteins, is one cause of the increase in the rates of cancers and cardiovascular diseases.

Moreover, animal proteins have many disadvantages, both in terms of their allergenicity, in particular with regards to proteins from milk and eggs, along with the degradation of our environment due to the intensive farming that is necessary for animal protein production.

Given this, manufacturers have gradually turned to alternative proteins to replace animal proteins. Indeed, it is known practice to use plant, insect or single cell proteins to replace all or some of the animal proteins in food products.

Single cell proteins are obtained by culturing microbial cells which will mainly convert sugars into proteins. After growing, proteins are directly recovered from culture media or after additional cell lysis step.

A first drawback of single cell proteins consists in their nucleic acid content. This content must be low because one of the end products of the metabolism of nucleic acids is uric acid which the human body, lacking the enzyme uricase required for catabolism thereof, is unable to degrade. Such technical problems have been already worked and some solutions already exist, mainly via chemical and/or physical hydrolysis or via enzymatic treatments with RNase.

Another drawback is the low functionality of single cell protein obtained. Indeed replacement of animal protein is not always easy, because the functional properties of single cell proteins are different from those of animal proteins. In this case functional properties refer to the physical or physicochemical properties, like emulsifying capacity, foaming capacity or especially gelling capacity, that will help to deliver the right sensory qualities to the food that will be made with.

Patents US 3,867,555 or US 3,848,812 disclose a method for producing low nucleic acids protein extracts via chemical and/or physical hydrolysis. After cell lysis, nucleic acids are hydrolyzed thanks to heat treatment at alkaline pH (e.g. about 9.5 to about 12.5 and a temperature of about 50°C to about 120°C for less than 4 hours in US 3,867,555). As it will be disclosed in the following chapters of application, such treatment at high pH and temperature will damage protein, e.g. by promoting Maillard reaction or hydrolysis, and will alter their functionalities including gelling capacity. Isoelectric precipitation which traditionally follows in order to recover single cell protein is also known to reduce protein functionalities including gelling capacity. Last point to mention, the protein content obtained via this method is also below 85%.

Patent application US 2022/0071231 discloses a method for producing low nucleic acids protein extracts via enzymatic hydrolysis with RNase. Such enzymatic treatment allows to reduce RNA content without heating too much at high pH. RNA content of single cell protein obtained is disclosed to be below 11%. As it will be exemplified in the following chapters of this application, if RNA is effectively reduced via this method, it will not be lower than 5%.

With such technical background, the purpose of the present invention is to overcome or reduce at least one of the disadvantages of the prior art, and/or to provide a single cell protein with low content of RNA, high functionalities including gelling capacity and high protein content.

## Brief Description of Drawings

### Figures

[Fig. 1] shows egg white protein after cooking in a frying pan.

[Fig. 2] shows SCP from Example 1 after cooking in a frying pan.

## General description of Embodiments

A first embodiment is a single cell protein characterized in that its content of protein is comprised between 80% and 99% expressed on dry matter of single cell protein, its gelling capacity measured with Test A is comprised between 15000 and 50000 and its content of RNA is comprised between 2% and 5% expressed on dry matter of single cell protein.

The first embodiment is preferably characterized in that it is a yeast protein.

The first embodiment is preferably characterized in that it is protein from *Saccharomyces cerevisiae.*

The first embodiment is preferably characterized in that its protein content is comprised between 85% and 97% expressed on dry matter of single cell protein.

The first embodiment is preferably characterized in that its gelling capacity measured with Test A is comprised between 20000 and 40000.

The first embodiment is preferably characterized in that its RNA content is comprised between 3% and 5% expressed on dry matter of single cell protein.

The first embodiment is preferably characterized in that its solubility measured with Test B from pH 4 to 8 is comprised between 60% and 99%.

The first embodiment is preferably characterized in that its solubility measured with Test B from pH 6 to 8 is comprised between 85% and 99%.

The first embodiment is preferably characterized in that its gelling capacity is comprised between 15000 and 25000, and its RNA content is comprised between 2% and 4%.

The first embodiment is preferably characterized in that its gelling capacity is comprised between 40000 and 50000, and its RNA content is comprised between 4% and 5%.

The first embodiment is preferably characterized in that said single cell protein is obtainable by a process according to the second embodiment.

A second embodiment is a process for producing a single cell protein comprising the steps of:

1. Producing a microorganism suspension containing proteins,

2. Lysing of the microorganism suspension produced in step 1, resulting in a lysate,

3. Microfiltration of the lysate obtained in step 2 with a permeability gradient membrane, resulting in a microfiltration permeate and a microfiltration retentate,

4. Ultrafiltration of the microfiltration permeate obtained in previous step 3, resulting in an ultrafiltration permeate and an ultrafiltration retentate,

5. Optional sterilizing of said ultrafiltration retentate obtained in previous step 4,

6. Optional drying of sterilized retentate obtained in previous step 5.

The second embodiment is preferably characterized in that the microorganism of step 1 is a yeast.

The second embodiment is preferably characterized in that the yeast is selected from the list of *Saccharomyces, Yarrowia, Hansenula, Candida,* and *Pichia.*

The second embodiment is preferably characterized in that crude protein content of microorganism is comprised between 40% and 60% expressed on dry matter of microorganism.

The second embodiment is preferably characterized in that step 1 includes a concentration step before step 2 selected from the list of centrifugation and press-filter in order to obtain a concentrated microorganism suspension with dry matter comprised between 10% and 30%. Before the concentration step, said dry matter content is usually below 10%.

The second embodiment is preferably characterized in that lysis of step 2 is performed using process selected from list of ball mill and high-pressure homogenization. At the end of step 2, a lysate is obtained.

The second embodiment is preferably characterized in that step 3 is done by succession of a centrifugation of the lysate obtained in previous step 2 resulting in an underflow and an overflow, followed by a microfiltration with a permeability gradient membrane of said overflow resulting in a microfiltration permeate and a microfiltration retentate.

The second embodiment is preferably characterized in that the centrifugation of step 3 produce an underflow which is recycled back in the microorganism suspension of step 2 to be lysed.

The second embodiment is preferably characterized in that pH of the lysate processed during permeability gradient microfiltration in step 3 is adjusted and regulated between 7,0 and 10,0.

The second embodiment is preferably characterized in that pH of the lysate processed during permeability gradient microfiltration in step 3 is adjusted and regulated between 8,0 and 9,5.

The second embodiment is preferably characterized in that microfiltration with a permeability gradient membrane of step 3 is done with a membrane having a porosity comprised between 0,1 microns and 0,2 microns, and at transmembrane pressure comprised between 0,50 bars and 1,50 bars.

The second embodiment is preferably characterized in that transmembrane pressure of microfiltration with a permeability gradient membrane of step 3 is comprised between 0,75 bars and 1,25 bars.

The second embodiment is preferably characterized in that ultrafiltration of step 4 is done with membrane porosity comprised between 3 to 10 KDa.

The second embodiment is preferably characterized in that sterilizing step of step 5 is done via a process selected in the list of pasteurization, HTST (High Temperature Short Time) pasteurization, ultraviolet treatment and filtration.

The second embodiment is preferably characterized in that drying step of step 6 is done via spray-drier.

The second embodiment is preferably characterized in that it consists only of all steps recited.

A third embodiment is the uses of said single cell protein from first embodiment or produced via process from second embodiment in several applications comprised in the list of food applications, feed applications, cosmetic applications and pharmaceutical applications.

The third embodiment is preferably selecting from the list of egg or egg white replacers in bakery, especially for producing angel cake, cream cake, and meringues.

The third embodiment is preferably for food applications as binders for meat or fish analogue.

The third embodiment is preferably for food applications as milk protein replacers in dairy (milk, yogurt, cheese production).


**Detailed description of Embodiments**


A first embodiment is a single cell protein characterized in that its content of protein is comprised between 80% and 99% expressed on dry matter of single cell protein, its gelling capacity measured with Test A is comprised between 15000 and 50000 and its content of RNA is comprised between 2% and 5% expressed on dry matter of single cell protein.

By "single cell protein", it is understood in the present application a protein that is produced by culture of microorganisms. Microorganisms can be microalgae, yeast, bacteria or fungi. Such single cell protein can be excreted by microorganisms or extracted after lysis. Single cell protein can also be referred in literature as "SCP".

In a specific embodiment, the microorganism is bacteria, and in a preferred embodiments strains are chosen among *Lactococcus lactis, Lactobacillus delbrueckii (bulgaricus), Lactobacillus helveticus, Lactobacillus pentosus, Lactobacillus plantarum, Lactobacillus curvatus, Lactobacillus casei, Lactobacillus salivarius, Pediococcus pentosaceus, Oenococcus oeni, Corynebacterium glutamicum, Cupriavidus necator, Brevibacillus agri, Xanthomonas campestris, Staphylococcus carnosus, Staphylococcus xylosus, Ideonella sakaiensis, Bacillus subtilis* and *Bacillus (Priestia) megaterium.*

By "protein", it is understood in the present application the molecules corresponding to a sequence of amino-acid residues. In this application, proteins are understood in their native form or in a modified form, including hydrolyzed proteins. In a specific embodiment of the invention, native form of the protein is preferred. These proteins can be used from compositions of different concentrations, including isolates having a protein content of above 80% on dry matter or concentrates having a protein content of between 50% and 80% on dry matter. In this application, isolates in which the protein content is above 80% on dry matter are particularly preferred.

The first embodiment is preferably characterized in that it is a yeast protein.

By "yeast", it is understood in the present application a single-cell microorganism belonging to the fungus kingdom. Yeasts are widely utilized in various industrial processes and biological research due to its ability to ferment sugars into alcohol and carbon dioxide through the process of anaerobic respiration. Such industrial processes include bakery, brewery, wine, ethanol, dairy and livestock feed industries.

Yeasts are widely studied since many years and following books offer deeper knowledge and definition:

- The Yeast Handbook Volume 2. Yeasts in Food and Beverages. Editors: Amparo Querol, Graham Fleet. 2006

- The Yeasts, Vol 5. Yeast technology. Eds: A.H. Rose, J.S. Harrison. 1993

- The yeast in the Brewery. G. Annemuller, H.J. Manger, P. Lietz. 2018

In a specific embodiment yeast types are chosen among *S. cerevisiae, Saccharomyces pastorianus, Saccharomyces carlsbergensis, Saccharomyces bayanus, Saccharomyces elipsoides, Saccharomyces uvarum, Saccharomyces ludwigii, Pichia pastoris (Komagataella), Candida utilis (Torula, Cyberlindnera jadinii)* and *Yarrowia (Candida) lipolytica.*

The first embodiment is preferably characterized in that it is a protein from *Saccharomyces cerevisiae.*

Any *Saccharomyces cerevisiae* can be used in the present application. More preferably, *Saccharomyces cerevisiae* are selected from public culture collections like DSM, ATCC for being produced by fermentation or commercially bought like Lal^Ferm® No1 from Lallemand.

The first embodiment is preferably characterized in that its protein content is comprised between 85% and 97% expressed on dry matter of single cell protein.

Protein content is measured by all conventional methods well known by a person skilled in the art.

More preferably, protein content is measured by quantifying nitrogen content using Kjeldahl or Dumas method. Preferably the nitrogen content is measured using Kjeldahl method and said obtained nitrogen content is multiplied by 6,25. Such method is described in Codex Guidelines on Nutrition Labelling CAC/GL 2-19851 or in EU Regulation 1169/2011. It corresponds to Test D. Obtained protein content is commonly known as "crude protein" content.

Dry matter is measured using any conventional methods known by a person skilled in the art. More preferably, analysis is done using moisture analyzer. Preferably, moisture analyzer is MX-50 from A&D. Principle of moisture analyzer it to weigh a sample (called W1), heating it in order to evaporate water until weight is stable and weighing it after heating (called W2). Dry matter is then calculated by dividing W2 by W1 and multiplying by 100. In this application, heating is done at 130°c until weight is stable.

The first embodiment is preferably characterized in that its gelling capacity measured with Test A is comprised between 20000 and 40000.

By "Gelling capacity", it is understood in the present application the functional property which corresponds to the capacity of a protein composition for forming a gel or a network, which increases the viscosity and generates a state of matter between the liquid and solid states. The term "gel strength" or "gel power" may also be used. To quantify this gelling capacity, it is thus necessary to generate this network and to evaluate its strength. To perform this quantification, in the present invention, Test A is used, the description of which is as follow.

The measurement of gelling capacity requires the measurement of viscoelastic properties, thus elastic modulus (G') is measured before and after gel setting. The elastic modulus is the modulus which characterizes solids. Thus, the gelling power is determined by the difference of G' before and after heat treatment which set gel.

Devices used for Test A are a balance having a precision of 0.1 mg such as Balance Mettler MS240S, a magnetic stirring plate and a rheometer such as DHR-2 from TA Instruments and MCR (Modular Compact Rheometer) 301 or 302 from Anton Paar.

Samples are prepared by solubilization of 15% of dry matter in water. 50g of solution are used to carry out the analysis (7.5g of protein for 42.5g of water).

1. Weigh the water (42.5g) in a glass vial,
2. Weigh the protein (7.5g),
3. Stir at around 350 rpm +/- 30 minutes on a stirring plate at room temperature. The samples are prepared 3 times, therefore, 3 measurements are made in order to calculate mean and standard deviation.

Gel measurement consists in 4 steps:

| Step 1: Frequency sweep | |
| --- | --- |
| Rheometers | DHR-2 or MCR 302 or 301 |
| Geometry | Concentric cylinders |
| Use of oil | No |
| Frequency | 10 to 0,02 Hz |
| Deformation | 0,2% |
| Temperature | 20°C |
| G'1 is measured. | |

| Step 2: Gelation | |
| --- | --- |
| Rheometers | DHR-2 or MCR 302 or 301 |
| Geometry | Concentric cylinders |
| Use of oil | No |
| Frequency | 1 Hz |
| Deformation | 0,2% |
| Temperature | From 20°C to 80°C in 2 hours, preferably at 0,5°C/minute |

| Step 3: Cooling | |
| --- | --- |
| Rheometers | DHR-2 or MCR 302 or 301 |
| Geometry | Concentric cylinders |
| Use of oil | No |
| Frequency | 1 Hz |
| Deformation | 0,2% |
| Temperature | From 80°C to 20°C at 2°C/minute |

| Step 4: Frequency sweep | |
| --- | --- |
| Rheometers | DHR-2 or MCR 302 or 301 |
| Geometry | Concentric cylinders |
| Use of oil | No |
| Frequency | 10 to 0,02 Hz |
| Deformation | 0,2% |
| Temperature | 20°C |
| G'2 is measured. | |

Gelling capacity (expressed in Pa) = G'2 - G'1.

Gelling capacity is preferably 20000, 20100, 20200, 20300, 20400, 20500, 20600, 20700, 20800, 20900, 21000, 21100, 21200, 21300, 21400, 21500, 21600, 21700, 21800, 21900, 22000, 22100, 22200, 22300, 22400, 22500, 22600, 22700, 22800, 22900, 23000, 23100, 23200, 23300, 23400, 23500, 23600, 23700, 23800, 23900, 24000, 24100, 24200, 24300, 24400, 24500, 24600, 24700, 24800, 24900, 25000, 25100, 25200, 25300, 25400, 25500, 25600, 25700, 25800, 25900, 26000, 26100, 26200, 26300, 26400, 26500, 26600, 26700, 26800, 26900, 27000, 27100, 27200, 27300, 27400, 27500, 27600, 27700, 27800, 27900, 28000, 28100, 28200, 28300, 28400, 28500, 28600, 28700, 28800, 28900, 29000, 29100, 29200, 29300, 29400, 29500, 29600, 29700, 29800, 29900, 30000, 30100, 30200, 30300, 30400, 30500, 30600, 30700, 30800, 30900, 31000, 31100, 31200, 31300, 31400, 31500, 31600, 31700, 31800, 31900, 32000, 32100, 32200, 32300, 32400, 32500, 32600, 32700, 32800, 32900, 33000, 33100, 33200, 33300, 33400, 33500, 33600, 33700, 33800, 33900, 34000, 34100, 34200, 34300, 34400, 34500, 34600, 34700, 34800, 34900, 35000, 35100, 35200, 35300, 35400, 35500, 35600, 35700, 35800, 35900, 36000, 36100, 36200, 36300, 36400, 36500, 36600, 36700, 36800, 36900, 37000, 37100, 37200, 37300, 37400, 37500, 37600, 37700, 37800, 37900, 38000, 38100, 38200, 38300, 38400, 38500, 38600, 38700, 38800, 38900, 39000, 39100, 39200, 39300, 39400, 39500, 39600, 39700, 39800, 39900, 40000, 40100, 40200, 40300, 40400, 40500, 40600, 40700, 40800, 40900, 41000, 41100, 41200, 41300, 41400, 41500, 41600, 41700, 41800, 41900, 42000, 42100, 42200, 42300, 42400, 42500, 42600, 42700, 42800, 42900, 43000, 43100, 43200, 43300, 43400, 43500, 43600, 43700, 43800, 43900, 44000, 44100, 44200, 44300, 44400, 44500, 44600, 44700, 44800, 44900, 45000, 45100, 45200, 45300, 45400, 45500, 45600, 45700, 45800, 45900, 46000, 46100, 46200, 46300, 46400, 46500, 46600, 46700, 46800, 46900, 47000, 47100, 47200, 47300, 47400, 47500, 47600, 47700, 47800, 47900, 48000, 48100, 48200, 48300, 48400, 48500, 48600, 48700, 48800, 48900, 49000, 49100, 49200, 49300, 49400, 49500, 49600, 49700, 49800, 49900, 50000and also ranges using previously cited values as borders.

By "RNA", it is understood in the present application ribonucleic acids of any molecular weight (MW): from high MW macromolecules packed in ribosomes to transfer RNA, matrix RNA, non-coding RNA, to monomeric nucleotides.

RNA content is preferably 2,0%, 2,1 %, 2,2%, 2,3%, 2,4%, 2,5%, 2,6%, 2,7%, 2,8%, 2,9%, 3,0%, 3,1%, 3,2%, 3,3%, 3,4%, 3,5%, 3,6%, 3,7%, 3,8%, 3,9%, 4,0%, 4,1%, 4,2%, 4,3%, 4,4%, 4,5%, 4,6%, 4,7%, 4,8%, 4,9%, 5,0% and also ranges using previously cited values as borders.

All methods for RNA determination well-known to a person skilled in the art can be used. A preferred method called Test C in this application was adapted according to Rut M. (1973) "Determination of nucleic acids on yeast and yeast related products." (Kvasny Prumysl, 19, 131-133). The samples were treated with 0.5M $HClO_4$ for 30 min at 90°C by mixing. Then, the samples were cooled and subsequently centrifuged at 17000 ×g for 5 min. The supernatants were neutralized with 1M NaOH and the amount of total RNA was determined by measuring the absorbance at 260 nm using a spectrophotometer, such as the NanoDrop 2000c spectrophotometer.

The first embodiment is preferably characterized in that its RNA content is comprised between 3% and 4% expressed on dry matter of single cell protein.

The first embodiment is preferably characterized in that its solubility measured with Test B from pH 4 to 8 is comprised between 60% and 99%.

The first embodiment is preferably characterized in that its solubility measured with Test B from pH 6 to 8 is comprised between 85% and 99%.

"Solubility" is understood to mean the quantification of the percentage of water-soluble material in a powder by diluting the powder in distilled water in controlled conditions especially temperature and pH, centrifuging the resulting suspension and analyzing the amount of solubilized material in the supernatant. In this application, the following Test B is preferred.

Test B: 150 g of distilled water are introduced into a 400ml beaker at 20°C +/-2°C by stirring with a magnetic stirrer bar, and precisely 5g of single cell protein sample to be tested are added. If required, the pH is adjusted to the desired value with 0.1 N NaOH. In this application preferred pH values for Test B are especially 4,0; 6,0 and 8,0. The content is supplemented with water to reach 200g of water. Mixing is carried out for 30 minutes at 1000 rpm and centrifugation is carried out for 15 minutes at 3000 g. 25g of the supernatant are collected and introduced into a crystallizing dish dried and tared beforehand. The crystallizing dish is placed in an oven at 103°C +/-2°C for 1 hour. It is then placed in a desiccator (with desiccant) to cool to ambient temperature. The sample is collected, then is weighed.

The solubility corresponds to the content of soluble dry matter, expressed as % by weight relative to the weight of the sample. The solubility is calculated with the following formula:

$$\frac{(m1 - m2) * (200 + P) * 100}{P1 * P}$$

In which:

- P = weight, in g, of the sample = 5 g
- m1 = weight, in g, of the crystallizing dish after drying
- m2 = weight, in g, of the empty crystallizing dish
- P1 = weight, in g, of the sample collected = 25 g

It is the merit of Applicant to have reach to produce a single cell protein, especially from yeast, that have protein content comprised between 80% and 99%, RNA content between 2% and 5% while having also gelling capacity between 15000 and 50000. Processes from prior art are incompatible to both produce such high gelling and protein content, while having such low RNA content. Treatment at high pH and temperature are well known to decrease RNA but, as it will be exemplified in the application below, it will also damage protein and alter their functionalities including gelling capacity. RNase or ribonucleases allow milder conditions for decreasing RNA but, as it will be also demonstrated below in the present application, RNA level lower than 5% are not reachable.

The first embodiment is preferably characterized in that its gelling capacity is comprised between 15000 and 25000, and its RNA content is comprised between 2% and 4%.

The first embodiment is preferably characterized in that its gelling capacity is comprised between 40000 and 50000, and its RNA content is comprised between 4% and 5%.

A second embodiment is a process for producing single cell protein comprising steps of:

1. Producing a microorganism suspension containing protein,

2. Lysis of the microorganism suspension produced in step 1, resulting in a lysate,

3. Microfiltration of the lysate obtained in step 2 with a permeability gradient membrane, resulting in a microfiltration permeate and a microfiltration retentate,

4. Ultrafiltration of the microfiltration permeate obtained in previous step 3, resulting in an ultrafiltration permeate and an ultrafiltration retentate,

5. Optional sterilizing of said ultrafiltration retentate obtained in previous step 4,

6. Optional drying of sterilized retentate obtained in previous step 5.

Step one of the second embodiment corresponds to the production of a microorganism suspension that contains proteins.

Relevant type of microorganisms has been already defined and discussed in the description part of first embodiment.

The second embodiment is preferably characterized in that the microorganism of step 1 is a yeast.

The second embodiment is preferably characterized in that yeast belongs to the genus *Saccharomyces,* such as *S. cerevisiae, S. pastorianus, S. elipsoides, S. ludwigii, S. chevalieri, S. boulardii, S. bayanus, S. italicus, S. delbrueckii, S. rosei, S. microellipsodes, S. carlsbergensis, S. bisporus, S. fermentati, S. rouxii,* or *S. uvarum;* a yeast belonging to the genus *Schizosaccharomyces,* such as *S. japonicus, S. kambucha, S. octosporus,* or *S. pombe;* a yeast belonging to the genus *Hansenula,* such as *H. wingei, H. arni, H. henricii, H. americana, H. canadiensis, H. capsulata,* or *H. polymorpha;* a yeast belonging to the genus *Candida,* such as *C. albicans, C. utilis, C. boidinii, C. stellatoidea, C. famata, C. tropicalis, C. glabrata,* or *C. parapsilosis;* a yeast belonging to the genus *Pichia,* such as *P. pastoris, P. kluyveri, P. polymorpha, P. barkeri, P. cactophila, P. rhodanensis, P. cecembensis, P. cephalocereana, P. eremophilia, P. fermentans,* or *P. kudriavzevii;* a yeast belonging to the genus *Kluyveromyces,* such as *K. marxianus;* a yeast belonging to the genus *Yarowia,* such as *Y. lipolytica* and a yeast belonging to the genus *Torulopsis,* such as *T. bovina,* or *T. glabrata.*

Production of microorganism suspension containing protein can be firstly done by cultivating a selected strain in a reactor, preferably a fermenter. Selected strains can be bought in a collection (e.g. ATCC, DSM) or screened in nature. Strains can be used as native or after being modified using GMO techniques like molecular biology tools or mutagenesis. Unique strain, mix of strains or even cocultivation of strains can be used.

Culture media and methods for growing microorganisms, preferably yeasts, are well known in the art. Suitable media comprise, for example, the YPD medium of Sigma Aldrich (Taufkirchen, Germany).

Microrganism suspension containing protein can secondly be obtained on the market, produced by commercial companies. In a preferred embodiment, microorganism suspension containing protein are byproducts left in the reactor after production of another main products like beer, ethanol, fuel, enzymes. Such products are mainly obtained in dried and/or compressed storage form. Water is added in order to obtain microorganism suspension containing proteins.

The second embodiment is preferably characterized in that crude protein content of microorganism is comprised between 40% and 60% expressed on dry matter of microorganism. Ways to measure the protein content was described in previous description of first embodiment above.

The second embodiment is preferably characterized in that step 1 includes a concentration step before step 2 selected from the list of centrifugation and press-filter in order to obtain a concentrated microorganism suspension with dry matter comprised between 10% and 30%. The dry matter percentages cited herein refer to wt. % based on the total weight of the suspension. The dry matter content of a suspension can be determined in accordance with standard procedures using commercially available devices, for example, the Moisture Analyzer MX-50 from A&D. Once the dry matter content of a starting suspension has been determined, this suspension can be adjusted to a pre-determined value either by diluting or concentrating the suspension.

Any relevant centrifuge apparatus based on separation of water and solid contents using sedimentation under gravity field can be used.

Any relevant filter based on separation of water and solid contents can be used. Press-filter is preferably used.

In a preferred embodiment, the cells (microorganisms) are washed with a buffer, such as water, before lysing step according to step two of second embodiment. Such washing step is useful to remove unwanted compounds from the microorganism suspension and/or reduce the taste of products obtained.

Step two of second embodiment corresponds to the lysis of the microorganism suspension containing proteins obtained in step one. Microorganisms commonly comprise inner soluble compounds (e.g. proteins, sugars) and insoluble compounds contained in a space called cytoplasm which is separated from external media via cell wall. Lysis consists of mechanical and/or chemical ways to break cell wall and release inner soluble compounds including proteins in order to make them available for next steps.

Yeast species like *Saccharomyces cerevisiae* have cell walls that mainly consists of β-glucans, mannoproteins, and chitin in a covalently linked matrix. Beneath the cell walls, yeast cells have a cell membrane of a lipid bilayer. To release proteins from the cell interior, both of these protective barriers need to be disrupted.

Lysis efficiency (LE) expressed in percentage is calculated based on the following protocol called Test C:

- lysate is centrifuged at 16639 G for 10min,

- dry matter & weight of underflow & overflow is measured

$$LE = (Qu \times DMu) / (Ql \times DMl) *100\%$$

Where:

- Ql - amount of lysate before centrifugation, expressed in g;

- DMl - dry matter of lysate, expressed in %.

- Qu - amount of underflow (intact yeast) after centrifugation, expressed in g;

- DMu - dry matter of underflow, expressed in %.

The second embodiment is preferably characterized in that lysis of step 2 is obtained using process preferably selected from list of ball mill, and high-pressure homogenization.

According to the process of the invention, cell lysis will be performed preferably mechanically, but use of other enzymes like e.g. glucanases or mannanases, which are commonly employed for yeast cell lysis, can be used if temperature does not exceed 40°C in order to keep protein functionalities.

In addition, microorganism cells contain a wide variety of proteases which are able to hydrolyze proteins into amino acids. When microorganism cells are disrupted, the proteases are released and could decompose the other protein molecules which are released by the microorganism cells. Accordingly, in a preferred embodiment of the invention, the cell lysis is performed at ambient temperatures. In this embodiment the activity of proteases is thus reduced.

According to a preferred embodiment, second step of second embodiment (*i.e.* the lysis) is performed at temperatures of below 40°C, more preferably below 30°C, and even more preferably below 20°C. A temperature of below 20°C, such as 15°C or 10°C, is particularly preferred to avoid an undesired decomposition of the proteins by proteases released from the microorganism cells. For precision, temperatures of 10°C, 11°C, 12°C, 13°C, 14°C, 15°C, 16°C, 17°C, 18°C, 19°C, 20°C, 21°C, 22°C, 23°C, 24°C, 25°C, 26°C, 27°C, 28°C, 29°C, 30°C, 31°C, 32°C, 33°C, 34°C, 35°C, 36°C, 37°C, 38°C, 39°C, 40°C and ranges obtained with these values as borders.

According to a preferred embodiment, microorganism cell lysis is achieved by use of a bead mill. On one hand, this ensures that the proteins remain native upon cell rupture. On the other hand, the use of a bead mill does not require any additives that could impair the food grade quality of the resulting protein compositions obtained. A bead mill normally comprises a chamber that is filled with beads which are moved around by a set of impeller fins. When a cell-containing suspension is passed through the chamber, the cells are disrupted upon collision with the beads. The efficiency of cell rupture can be adjusted by routine measures, for example, by changing the flow rate which determines how fast the microorganism suspension (such as yeast suspension) is passed through the chamber of the bead mill. Normally, a low flow rate leads to high lysis efficiency, since the cells have more time to collide with the grinding beads. Dependent on the volume of the chamber of the bead mill, flow rates of at least 10 kg/h can be selected, such at least 20 kg/h, at least 50 kg/h, or at least 100 kg/h. It is particularly preferred that during operation the bead mill is cooled to temperatures below 20°C, such 18°C, 16°C, 14°C, 12°C or 10°C. Bead mills are offered by different manufacturers, for example, the Dyno®-Mill Multi Lab Wab.

Another factor that has a direct impact on the efficiency of lysis is the bead material and size. Beads can be made of different materials, e.g. glass, ceramic or plastic. Particularly good results have been achieved with zirconium oxide beads. In addition, these beads are significantly more durable compared to glass beads. The beads used for disrupting the yeast cells, e.g. the zirconium oxide beads, may have different sizes which normally range from 0.2-2.0 mm. It has been found that for disrupting the yeast cells, a bead size of 0.25-0.35 mm leads to particularly good results. Accordingly, a bead size of 0.25-0.35 mm is particular preferred. Bead filling volumes of between 30-80% may be used, for example, about 30%, about 35%, about 40%, about 45%, about 50%, about 55%, about 60%, about 65%, about 70%, or about 75%. A bead filling volume of between 50-60% is particularly preferred according to the invention.

Another way of influencing the efficiency of cell rupture is to adapt the impeller architecture. The impeller is normally mounted with plastic fins to move the beads. For improved cell rupture, it is for example possible to provide accelerators onto the rotor. Accelerators are designed to force the beads to collide more often. The impeller rotation speed is another parameter that may be adjusted to improve cell rupture. A rotor speed of between 1-20 m/s can be used, for example, about 1 m/s, about 2 m/s, about 3 m/s, about 4 m/s, about 5 m/s, about 6 m/s, about 7 m/s, about 8 m/s, about 9 m/s, about 10 m/s, about 11 m/s, about 12 m/s, about 13 m/s, about 14 m/s, about 15 m/s, about 16 m/s, about 17 m/s, about 18 m/s, or about 19 m/s. A skilled person would be readily able by routine experimentation to find optimum parameters for disrupting a yeast suspension in a bead mill.

Yet another way of mechanically lysing the microorganism cells uses high-pressure homogenization (HPH). High pressure homogenization is a method commonly used in the pharmaceutical, chemical and food industry to stabilize emulsions, and the like. It can however also be used to disrupt bacteria and yeast in order to extract intracellular products from the cells. During HPH, the cells to be disrupted are passed through a narrow slit under high pressure. As the yeast cells pass this narrow slit, the flow rate sharply increases and the pressure abruptly decreases. The resulting shear forces cause cell disruption.

HPH devices for use in the method of the present invention can be obtained from different manufacturers. For example, the EmulsiFlex-C3 of Avestin Europe GmbH (Mannheim, Germany) or the 1000/2000 Homogenizer of SPX Flow Technology Germany GmbH (Moers, Germany) can be used. The microorganism suspension can be passed through the HPH device one time or several times. If the suspension is passed through the device several times, the efficiency of cell rupture increases. The pressure of the device can be set to a pressure of at least 700 bar. Preferably, the pressure will be at least 1000 bar, at least 1100 bar, at least 1200 bar, at least 1300 bar, at least 1400 bar, at least 1500 bar, at least 1600 bar, at least 1700 bar, at least 1800 bar, at least 1900 bar, or at least 2000 bar or more.

Microfluidics Microfluidizer 7250-10 equipment is preferred for cell lysis.

After lysis of the cells as described above, the pH of the lysate may approach a value of 6.0 or below. It is preferred that the pH of the lysate is readjusted to a value between 7.0 and 10.0. For example, the pH of the lysate is readjusted to about 7,0; 7,1; 7,2; 7,3; 7,4; 7,5; 7,6; 7,7; 7,8; 7,9; 8,0; 8,1; 8,2; 8,3; 8,4; 8,5; 8,6; 8,7; 8,8; 8,9; 9,0; 9,1; 9,2; 9,3; 9,4; 9,5; 9,6; 9,7; 9,8; 9,9; 10,0 and all ranges that can be obtained with these values as borders. More preferably, pH will be adjusted between 8,5 and 9,5.

Step three of second embodiment corresponds to a microfiltration of the lysate obtained in step 2 with a permeability gradient membrane, resulting in a microfiltration permeate and a microfiltration retentate.

As it will be described below, the microfiltration with a permeability gradient membrane is key in this step in order to separate soluble proteins (containing the functional proteins) in its permeate on one side and insoluble proteins containing main part of RNA in its retentate in the other side.

Microfiltration is a type of physical filtration process where a fluid is passed through a pore-sized membrane filter to separate suspended particles from process liquid. Microfiltration usually serves as a pre-treatment for other separation processes such as ultrafiltration, and a post-treatment for granular media filtration. In terms of approximate molecular weight these membranes can separate macromolecules of molecular weights generally less than 100,000 g/mol.

The fluid is passed through at a relatively high velocity, parallel or tangential to the membrane in a sheet or tubular form. A pump is commonly fitted onto the processing equipment to allow the fluid to pass through the membrane filter. There are also two pump configurations, either pressure driven or vacuum. A differential or regular pressure gauge is commonly attached to measure the pressure drop between the outlet and inlet streams.

Microfiltration in the present application use a particular membrane which is commonly called "permeability gradient" or "permeability gradient membrane". That is why the microfiltration according to the present disclosure is called a "microfiltration with a membrane having a permeability gradient" or a "permeability gradient microfiltration".

Permeability gradient membrane is designed for optimizing compounds transfer across the microfiltration membrane. In conventional microfiltration membranes, the natural pressure drop creates asymmetric transmembrane pressure (TMP) from the inlet to the outlet of the flow channel. Permeability gradient membranes are concepted with permeability gradient built into the support structure which allows a stable microfiltration regime all along the membrane. Such membranes are well known in industry. Pall® Membralox® GP is an example of commercial membrane suitable for application. Applicant has surprisingly demonstrated that microfiltration membranes with a permeability gradient allows to retain efficiently RNA in retentate while allowing purified soluble protein to pass in filtrate.

The second embodiment is preferably characterized in that step 3 is done by the succession of a centrifugation of the lysate obtained in previous step 2, resulting in a centrifugation underflow and a centrifugation overflow, followed by a microfiltration with a permeability gradient membrane of said overflow resulting in a microfiltration permeate and a microfiltration retentate.

Said centrifugation overflow is free of intact cell or cell wall debris, preferably less than 1%, more preferably less than 0,5%, more preferably less than 0,1%.

The second embodiment is preferably characterized in that the centrifugation of step 3 produces an underflow which is recycled back in lysis of step 2. Said underflow contains intact cells and/or cell wall debris and can be recycled back in the microorganism suspension of step 2, to be lysed, therefore improving the yield.

The second embodiment is preferably characterized in that pH of the lysate obtained in step 2 is adjusted and regulated between 7,0 and 10,0 while feeding microfiltration of step 3.

The second embodiment is preferably characterized in that pH of the lysate obtained in step 2 is adjusted and regulated between 8,0 and 9,5 while feeding microfiltration of step 3.

As it will be exemplified, precise control of pH during during the microfiltration with a permeability gradient membrane will allow to optimize RNA separation between permeate and retentate.

pH can be adjusted with any of well-known reagents selecting in basic and acid reagents. In a preferred embodiment, NaOH, KOH and their blend will be used as basic reagents. In a preferred embodiment, HCl, $H_2SO_4$ and their blend will be used as acid reagents.

The second embodiment is preferably characterized in that permeability gradient microfiltration of step 3 is done with a membrane having a porosity comprised between 0,1 microns and 0,2 microns, at transmembrane pressure comprised between 0,50 bars and 1,50 bars.

The second embodiment is preferably characterized in that transmembrane pressure is comprised between 0,75 bars and 1,25 bars.

Microfiltration membrane porosity can be 0,10; 0,11; 0,12; 0,13; 0,14; 0,15; 0,16; 0,17; 0,18; 0,19; 0,20 microns and all ranges that can be obtained with these values as borders.

Transmembrane pressure (TMP) is to be understood as the pressure difference between two sides of membranes. This TMP is the power allowing membrane filtration.

Transmembrane pressure can be 0,50; 0,55; 0,60; 0,65; 0,70; 0,75; 0,80; 0,85; 0,90; 0,95; 1,00; 1,05; 1,10; 1,15; 1,20; 1,25; 1,30, 1,35; 1,40; 1,45; 1,50 bars and all ranges that can be obtained with these values as borders.

In a preferred embodiment, the permeability gradient microfiltration is carried out in a tangential filtration module.

In an even more preferred embodiment, said microfiltration is followed by a diafiltration step. In this embodiment, diafiltration corresponds to successive steps of concentration of microfiltration retentate and addition of water to increase the purity of retentate. Preferably, diafiltration will be carried out one, two or three times with one, two or three volumes of water. More preferably, diafiltration will be carried out one time with three volumes of water.

Microfiltration retentate obtained in step 3 can also undertake a separation process that will allow production of a protein concentrate. Such additional step allows to maximize overall yield by proposing also a protein concentrate and a composition containing RNA products (including inosinate and guanylate as well as others ribotides).

Step 4 corresponds to an ultrafiltration of the permeate obtained with a permeability gradient microfiltration of step 3. The obtained ultrafiltration retentate contains single cell proteins.

Ultrafiltration refers to the membrane separation method, which is distinguished from microfiltration or nanofiltration by the size of suspended or solution particles that may pass through. For ultrafiltration this size is between 1 and 100 nanometers (nm).

The second embodiment is preferably characterized in that the ultrafiltration of step 4 is done with a membrane porosity comprised between 3 to 10 KDa.

The cutoff thresholds can therefore be 3 KDa, 4 KDa, 5 KDa, 6 KDa, 7 KDa, 8 KDa, 9 KDa and 10 KDa, as well as all the ranges formed by these values. The preferred filtration temperature is 5°C to 15°C. The filtration temperature can therefore be 5°C, 6°C, 7°C, 8°C, 9°C, 10°C, 11°C, 12°C, 13°C, 14°C, 15°C, and all ranges formed by these values.

Preferably, the transmembrane pressure will be between 1 and 10 bars, preferably between 2 and 4 bars. The transmembrane pressure values may be 1 bar, 2 bars, 3 bars or 4 bars, as well as all the ranges formed by these values. The transmembrane pressure is a parameter well known to the skilled person which consists of the pressure difference on either side of the ultrafiltration membrane.

In a preferred embodiment, said ultrafiltration is carried out in a tangential filtration module.

In an even more preferred embodiment, said ultrafiltration is followed by a diafiltration step. In this embodiment, diafiltration corresponds to successive steps of concentration of ultrafiltration retentate and addition of water to increase the purity of retentate. Preferably, diafiltration will be carried out in order to achieve at least a protein richness of 70%. Preferably, diafiltration will be carried out in order to achieve at least a protein richness of 80%. Protein richness can be 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99%, 100%, and all ranges formed by these values.

Ultrafiltration permeate can also be highly valorized as it contains, especially in case of yeast as starting material, yeast peptides that confers kokumi taste. Kokumi is predominantly found in the realm of Japanese cuisine, where its taste sensation occurs naturally in fermented foods like alcohol, soy sauce, fish sauces and shrimp paste. Ultrafiltration permeate can allow easy use of protein product to confer kokumi taste in food applications, without use of fermentation.

The second embodiment is preferably characterized in that sterilizing step of step 5 is done via a process selected in the list of pasteurization, HTST, ultraviolet treatment and filtration.

Preferably, the process may then include an additional heat treatment of ultrafiltration retentate. Temperature and time conditions can vary widely in this step, for example from 70 to 140°C and from 0.1 seconds to several minutes. Main driver will be to allow reduction of microorganism without altering protein functionalities. According to a first variant of this additional heat treatment step, the temperature ranges from 70 to 90°C and its duration ranges from 0.1 seconds to 30 minutes. According to a second variant of this additional heat treatment step, the temperature ranges from 90 to 110°C and its duration ranges from 0.1 seconds to 5 minutes. In another variant, this additional heat treatment step is performed at a temperature ranging from 110 to 140°C for a time ranging from 0.1 to 30 seconds, preferably from 0.2 to 15 seconds, for example from 0.3 to 10 seconds. This step may aim to sanitize the single cell protein, preferably from yeast. To achieve this additional heat treatment step, single cell protein, preferably from yeast can be in the form of an aqueous dispersion, preferentially having a dry matter ranging from 10 to 25%, for example from 15 to 20%. Advantageously, following heat treatment step, a cooling step may occur. According to a preferred variant, this cooling step is obtained by rapid cooling (≪flash-cooling≫). At the end of this step, the temperature can range from 60 to 100°C, for example between 70 and 90°C. Similarly, this rapid cooling step ("flash-cooling") is performed by applying a vacuum to the aqueous dispersion of single cell protein, preferably yeast, the vacuum applied being determined according to the chosen cooling temperature.

The second embodiment is preferably characterized in that drying step of step 6 is done via a spray-drier.

The principle of spray drying is based on the dispersion of a solution into fine droplets which are introduced into a flow of hot air. The solvent evaporates from the substrate droplets so that dry product clusters remain. Spray drying is usually performed at temperatures that are normally higher than the melting temperature of proteins. Standard spray drying devices can be used, such as the Mini Spray Dryer B-290 from Büchi Labortechnik GmbH (Essen, Germany) or the Mobile Minor™ Spray Dryer from GEA (Berlin, Germany).

Freeze drying or lyophilization is a process which removes water from a product to extend shelf life. Freeze drying encompasses freezing the product, reducing the pressure and adding heat to allow the frozen water in the material to sublimate. Various methods can be applied for freezing the product. For example, freezing can be achieved by using a standard freezer or a chilled bath. Cooling the product below its triple point ensures that sublimation will occur upon heating. To prevent the formation of large crystals that may damage the structure of the product to be dried, freezing is done rapidly. About 95% of the water in the product is removed when the frozen water sublimates. Most materials can be dried to 1-5% residual moisture. Standard freeze-drying devices can be used, such as the Lyovac™ devices from GEA (Berlin, Germany), the Gamma 2-20 Freeze dryer LCM-1 from Christ (Osterode am Harz, Germany), or the Christ Martin™ Alpha 1-2 Lyophilisator from Fisher Scientific GmbH (Schwerte, Germany).

The second embodiment is preferably characterized in that it consists only of all steps claimed.

In a preferred embodiment, the second embodiment is a process for producing a single cell protein consisting in the following steps of:

1. Producing a microorganism suspension containing protein,

2. Lysing of the microorganism suspension produced in step 1, resulting in a lysate.

3. Microfiltration of the lysate obtained in step 2 with a permeability gradient membrane, resulting in a microfiltration permeate and a microfiltration retentate,

4. Ultrafiltration of the microfiltration permeate obtained in previous step 3, resulting in an ultrafiltration permeate and an ultrafiltration retentate,

5. Optional sterilizing of said ultrafiltration retentate obtained in previous step 4,

6. Optional drying of sterilized retentate obtained in previous step 5.

It is a considerable advantage of the process to allow a production of a functional isolate, but potentially also a protein concentrate, RNA products and a kokumi peptide starting from a unique microorganism. Such multiple products allow a better valorization.

A third embodiment is uses of single cell protein as described above or produced via a process as described above in industrial applications comprised in the list of food applications, feed applications, cosmetic applications and pharmaceutical applications.

The food and feed industry products are understood to mean industrial confectionery (for example, chocolate, caramel, jelly sweets), bakery products (for example, bread, brioches, muffins), the meat and fish substitutes industry (for example, sausages, hamburgers, fish nuggets, chicken nuggets), sauces substitutes (for example, bolognaise, mayonnaise), substitutes of products derived from milk (for example, cheese, plant milk), beverages (for example, high protein beverages, powdered beverages to be reconstituted).

The invention will be of particular interest in the field of analogs of meat, fish, sauces, soups. A particular application relates to the use of the composition according to the invention for manufacturing meat substitutes, in particular minced meat, and also bolognaise sauce, steak for hamburgers, meat for tacos and pitta, "Chili sin carne".

The third embodiment is preferably selecting from the list of egg or egg white replacers in bakery especially for producing angel food cake, cream cake, meringues.

The third embodiment is preferably useful for producing binders for meat or fish analog.

The third embodiment is preferably for food as milk protein replacers in dairy (milk, yogurt, cheese production).

Invention will be better understood by reading of following non-exhaustive examples.

**Examples**

**Example 1: Process of the invention**

Commercial bakery yeast (*S. cerevisiae*) Lal^Ferm® No1 from Lallemand is used to produce a suspension in water in a range of 17% DM (dry matter).

The suspension is transferred to a high-pressure homogenizer Microfluidics Microfluidizer® 7250-10 working at least at 600 bar and cooled in order not to exceed 17°C in the suspension. Recirculation through the homogenizer is carried out to reach at least 70% of disrupted cells, as measured by Test C described in description above.

Suspension with disrupted cells (a lysate) is transferred to a microfiltration (MF) system comprising ceramic membranes having a permeability gradient (such as Pall Membralox®) of 0.1 μm pore size installed for separation of the suspension into permeate (called MF-P) and retentate (called MF-R). After microfiltration is completed, diafiltration with 3 volume water is carried out and a diapermeate (MF_DP) and a diaretentate (MF-DR) are obtained. MF-P is mixed with MF-DP and is transferred to the next step of ultrafiltration.

During all microfiltration the transmembrane pressure is maintained at 1 bar; pH is maintained in the MF system at 8.5 with controlled addition of NaOH 2% solution; the temperature is maintained in the range of 18-22°C.

Mixed permeates from MF (MF-P plus MF-DP) undergo protein concentration and purification in an ultrafiltration step. 5 kDa hollow-fiber membranes Fluid Systems Romicon 6" Hollow Fiber PM 5 from Koch are used. Ultrafiltration (UF) process is carried out at 4-8°C temperature to restrain proteolysis by yeast endogenous proteases. The retentate obtained at the end of the UF process (after concentration from 900L until 20L) is diafiltered in the same UF system by water (3 volume). The obtained UF diaretentate as a purified protein concentrate may be transferred for a sterilization step or directly to drying by a spray dryer.

Spray-dryer is operated at 200°C air temperature at an inlet and 70°C at an outlet, with 0.25 bar pressure.

Product obtained is called "Product of the invention" or "SCP of Example 1".

## Example 2: Prior art process with heat and alkali treatment to hydrolyze RNA

Example 2 aims to reproduce Example 1 of US 3,867,555. Yeast suspension was prepared, homogenized, and centrifuged as recommended in Example 1 of US 3,867,555 in order to produce an alkali extract. Then the alkali extract was adjusted to pH 12 by the addition of 10 N NaOH. The extract was incubated for 1 hour at 60°C with gentle agitation. Then pH was adjusted to 4.5 with 85% phosphoric acid and filtered. The filtrate was washed and spray-dried.

Product obtained is called "Prior art product #1"

## Example 3: Prior art process with RNase

Example 3 aims to reproduce Example 1 from US2022/0071231 A1, combined with preferred use of RNase teached in paragraph 23 of this application US2022/0071231.

*Saccharomyces cerevisiae* yeast cells were suspended in water at 14% dry matter content and pH 7.5 using NaOH. Yeast cells were then lysed using a Dyno®-Mill Multi Lab Wab using yttria-stabilized zirconium oxide beads with a size of 0.25-0.35 mm. The flow rate was set to 7 kg/h and the rotor speed to 8 m/s. The bead filling volume was set to 65%. The pH of the lysate obtained from the bead mill was adjusted to 7.6 using NaOH. Subsequently, the lysate was subjected to centrifugation for 120 minutes at 25000g. The supernatant was homogenized, treated by RNase A obtained from Thermo Fisher Scientific used at the recommended dosage of 0.01% for 30 min at 8°C with gentle agitation. Supernatant treated by RNase was then subjected to activated carbon filtration using an active carbon filtration cartridge filled with steam activated Norit® SX Plus. The solution obtained from activated carbon filtration was sterilized at a temperature of 130°C for 3 seconds and subsequently spray-dried.

RNA level originally assessed to 12% is reduced to 8,5% which indicates that RNase treatment was active.

Product obtained is called "Prior art product #2"

## Example 4: Influence of pH on gradient MF

Example 1 is reproduced but pH of feed of permeability gradient microfiltration is adjusted and controlled at pH 5,5 and pH 11.

Products obtained are called "Comparative product #1 (pH 5,5) and #2 (pH 11)".

## Example 5: Summary of all previous examples to compare performances

|  | Product of the invention | Prior art product #1 | Prior art product #2 | Comparative product #1 | Comparative product #2 |
|---|---|---|---|---|---|
| DM (%) | 92.0 | 92.0 | 93.0 | 94.0 | 92.4 |
| Protein (expressed in %/DM, using Test D) | 91.6 | 78.0 | 79.0 | 91.0 | 94.6 |

(continued)

|  | Product of the invention | Prior art product #1 | Prior art product #2 | Comparative product #1 | Comparative product #2 |
|---|---|---|---|---|---|
| RNA, expressed in %/DM, using Test C | 3.5 | 1.8 | 8.5 | 0.9 | 7.3 |
| Gelling capacity, expressed in Pa, using Test A | 24000 | <500 | 9000 | <500 | Impossible to measure, the suspension is gelling at room temperature |

## Example 6: Comparison of functional properties between egg white protein and SCP from invention

First, the minimal concentration leading to coagulation has been evaluated, using following procedure:

- 10%, 7,5%, 5% and 2,5% protein solution were prepared (at iso protein content between SCP protein from the invention and egg white protein)

- 10g of the solutions are put in test tube and place in water bath at 80°C

- Observations made were:

  ○ Coagulation: Yes or no

  ○ Time to coagulate

Results are:

|  | Egg white protein (control) | SCP from example 1 |
|---|---|---|
| 10% | 3 min 30 | 2 min 30 |
| 7,5% | 3 min 30 | 3 min 30 |
| 5% | 4 min | 4 min |
| 2,5% | No coagulation | No coagulation |

Results show that minimal concentration leading to coagulation is the same (5%).

As second comparison, behavior in a frying pan was observed. 80g of the 10% solution used for coagulation test is heated in a pan to observe coagulation capacity. The idea of this simplified test is to imitate the making of an omelet.

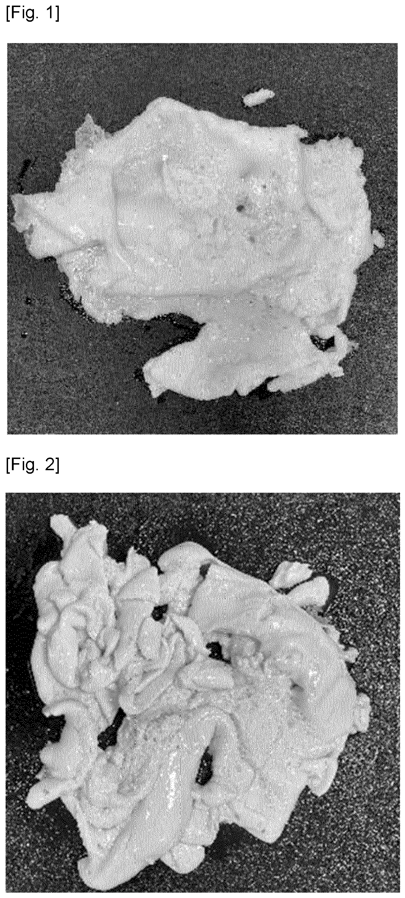

Figure 1 shows the results with egg white protein and Figure 2 shows the results using SCP from Example 1. External aspect is very close. After consumption by 10 people, 8 on 10 found SCP (from Example 1) very close to egg white protein.

## Example 7: Use of SCP from invention in angel food cake

Angel food cake is an interesting application to highlight how single-cell protein of the invention properties are close to egg white ones. The quantity of egg white used in the recipe is important compared to other ingredients.

Recipes are the following ones:

| Weights in g | Control angel food cake (with egg white proteins) | Angel food cake (with SCP of the invention) |
|---|---|---|
| Wheat flour | 86 | 86 |
| Sucrose | 200 | 200 |
| Salt | 1 | 1 |

(continued)

| Weights in g | Control angel food cake (with egg white proteins) | Angel food cake (with SCP of the invention) |
|---|---|---|
| Water | 206 | 206 |
| Baking powder | 3 | 3 |
| Egg white powder | 44 | 0 |
| SCP from Example 1 | 0 | 44 |

Process is:

Mix, in Hobart planetary mixer egg white protein or SCP from Example 1 with water, at speed 9 during 3 minutes,

Add slowly sucrose during 30 seconds and continue to whip during 1'30 minutes,

Add other ingredients slowly so as not to break the foam,

Put in a fairly high mold,

Heat in the oven, at 170°C for 30 minutes.

After consumption by 10 people, everyone found that both cakes are very close.

**Example 8: Use of SCP from invention in cream cake**

Cream cake is also an interesting application to highlight how single-cell protein of the invention properties are close to egg white ones.

Recipes are the following ones:

| Weights in g | Control cream cake (with egg white proteins) | Inventive cream cake (with SCP of the invention) |
|---|---|---|
| Wheat flour | 27,5 | 27,5 |
| Sucrose | 24,5 | 24,5 |
| Salt | 0,2 | 0,2 |
| Water | 12 | 12 |
| Baking powder | 1,3 | 1,3 |
| Corn oil | 16 | 16 |
| Egg white powder | 18,5 | 0 |
| SCP from Example 1 | 0 | 18,5 |

Process is:

- Whip protein, sucrose and water for 5 minutes at speed 3 in a planetary mixer (Hobart type),

- Add other ingredients, mix for 1 minute at speed 2,

- Add oil, and mix for 1 min speed 2,

- Put in a greased aluminum mold (300g),

- Bake in an oven at 170°C for 40 minutes.

**Claims**

1.  Single cell protein **characterized in that** its content of protein is comprised between 80% and 99% expressed on dry matter of single cell protein, its gelling capacity measured with Test A is comprised between 15000 and 50000 and its content of RNA is comprised between 2% and 5% expressed on dry matter of single cell protein.

2.  Single cell protein from claim 1 **characterized in that** it is a yeast protein.

3.  Single cell protein from claim 2 **characterized in that** it is a protein from *Saccharomyces cerevisiae.*

4.  Single cell protein from anyone of claims 1 to 3 **characterized in that** its protein content is comprised between 85% and 97% expressed on dry matter of single cell protein.

5.  Single cell protein from anyone of claims 1 to 4 **characterized in that** its solubility measured with Test B from pH 4 to 8 is comprised between 60% and 99%.

6.  Single cell protein from anyone of claims 1 to 5 **characterized in that** its gelling capacity is comprised between 15000 and 25000, and its RNA content is comprised between 2% and 4%.

7.  Process for producing a single cell protein comprising steps of:

    1. Producing a microorganism suspension containing protein,
    2. Lysing of the microorganism suspension produced in step 1, resulting in a lysate,
    3. Microfiltration of the lysate obtained in step 2 with a permeability gradient membrane, resulting in a microfiltration permeate and a microfiltration retentate,
    4. Ultrafiltration of the microfiltration permeate obtained in previous step 3, resulting in an ultrafiltration permeate and an ultrafiltration retentate,
    5. Optional sterilizing of retentate obtained in previous step 4,
    6. Optional drying of sterilized retentate obtained in previous step 5.

8.  Process of claim 7 **characterized in that** the microorganism of step 1 is a yeast.

9.  Process of anyone of claims 7 to 8 **characterized in that** crude protein content of microorganism is comprised between 40% and 60% expressed on dry matter of microorganism.

10. Process of anyone of claims 7 to 9 **characterized in that** step 1 includes a concentration step before step 2 selected from the list of centrifugation and press-filter in order to obtain a concentrated microorganism suspension with dry matter comprised between 10% and 30%.

11. Process of anyone of claims 7 to 10 **characterized in that** step 3 is done by succession of a centrifugation of the lysate obtained in previous step 2, resulting in an underflow and an overflow, followed by a permeability gradient microfiltration of said overflow resulting in a microfiltration permeate and a microfiltration retentate.

12. Process of anyone of claims 7 to 11 **characterized in that** said centrifugation of step 3 produces an underflow which is recycled back in the microorganism suspension of step 2.

13. Process of anyone of claims 7 to 12 **characterized in that** pH of the lysate obtained in step 2 is adjusted and regulated between 7,0 and 10,0.

14. Process of anyone of claims 7 to 13 **characterized in that** the microfiltration of step 3 is done with a membrane having a porosity comprised between 0,1 microns and 0,2 microns, at transmembrane pressure comprised between 0,50 bars and 1,50 bars.

15. Process of anyone of claims 7 to 14 **characterized in that** the ultrafiltration of step 4 is done with a membrane having a porosity comprised between 3 to 10 KDa.

16. Process of anyone of claims 7 to 15 **characterized in that** it consists only of all steps claimed.

17. Uses of a single cell protein from claim 1 to 6 or produced via a process from claims 7 to 16 for food applications, feed applications, cosmetic applications or pharmaceutical applications.

[Fig. 1]

[Fig. 2]

Europäisches
Patentamt

European
Patent Office

Office européen
des brevets

# EUROPEAN SEARCH REPORT

**Application Number**

EP 24 30 5771

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2023/227681 A1 (PROTEINDISTILLERY GMBH [DE]) 30 November 2023 (2023-11-30) <br> * claim 4 * <br> * claim 9 * <br> * claim 13 * <br> ----- | 1-6,17 | INV. <br> A23J1/18 <br> A23J3/20 <br> A23L31/15 <br> A23L33/145 <br> A23L33/195 |
| X | US 3 887 431 A (ROBBINS ERNEST ALECK ET AL) 3 June 1975 (1975-06-03) <br> * claim 1 * <br> * claim 5 * <br> * claim 15 * <br> * column 19, line 36 - line 40 * <br> ----- | 1-6,17 | B01D61/14 <br> B01D61/58 <br> C07K1/34 <br> C12N1/06 <br> C12N1/08 |
| X | US 2020/086272 A1 (NTOKOU ELENI [DK] ET AL) 19 March 2020 (2020-03-19) <br> * claim 32 * <br> * claim 33 * <br> * claim 34 * <br> * claim 35 * <br> * paragraph [0137] * <br> * paragraph [0140] * <br> * paragraph [0151] * <br> ----- | 1,4-7, 12,15-17 | |
| X | EP 4 119 654 A1 (YEASTUP AG [CH]) 18 January 2023 (2023-01-18) <br> * claim 1 * <br> * claim 2 * <br> * claim 14 * <br> * paragraph [0026] * <br> * paragraph [0098] * <br> ----- | 7-15 | TECHNICAL FIELDS SEARCHED (IPC) <br><br> A23J <br> C07K <br> B01D <br> C12R <br> A23L <br> C12N |
| X | US 2022/087286 A1 (HENDERSON JR CARL ALLEN [US] ET AL) 24 March 2022 (2022-03-24) <br> * paragraph [0082] * <br> * paragraph [0283] - paragraph [0288] * <br> * paragraph [0304] - paragraph [0306] * <br> ----- | 7,8,12, 15 | |

The present search report has been drawn up for all claims

| Place of search | Date of completion of the search | Examiner |
|---|---|---|
| The Hague | 11 October 2024 | Alonso Martínez, D |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P4C01)

## ANNEX TO THE EUROPEAN SEARCH REPORT
## ON EUROPEAN PATENT APPLICATION NO.

EP 24 30 5771

This annex lists the patent family members relating to the patent documents cited in the above-mentioned European search report.
The members are as contained in the European Patent Office EDP file on
The European Patent Office is in no way liable for these particulars which are merely given for the purpose of information.

11-10-2024

| Patent document cited in search report | | Publication date | Patent family member(s) | | Publication date |
|---|---|---|---|---|---|
| WO 2023227681 | A1 | 30-11-2023 | AU | 2023275927 A1 | 03-10-2024 |
| | | | WO | 2023227681 A1 | 30-11-2023 |
| US 3887431 | A | 03-06-1975 | CA | 1025270 A | 31-01-1978 |
| | | | US | 3887431 A | 03-06-1975 |
| US 2020086272 | A1 | 19-03-2020 | BR | 112019012705 A2 | 19-11-2019 |
| | | | CA | 3047355 A1 | 28-06-2018 |
| | | | CN | 110087478 A | 02-08-2019 |
| | | | EP | 3558025 A1 | 30-10-2019 |
| | | | JP | 2020501547 A | 23-01-2020 |
| | | | RU | 2019122797 A | 22-01-2021 |
| | | | US | 2020086272 A1 | 19-03-2020 |
| | | | WO | 2018115042 A1 | 28-06-2018 |
| EP 4119654 | A1 | 18-01-2023 | CN | 117940553 A | 26-04-2024 |
| | | | EP | 4119654 A1 | 18-01-2023 |
| | | | EP | 4370650 A1 | 22-05-2024 |
| | | | JP | 2024525667 A | 12-07-2024 |
| | | | WO | 2023285480 A1 | 19-01-2023 |
| US 2022087286 | A1 | 24-03-2022 | US | 2022087286 A1 | 24-03-2022 |
| | | | US | 2024292865 A1 | 05-09-2024 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 3867555 A
- US 3848812 A
- US 20220071231 A
- US 20220071231 A1

**Non-patent literature cited in the description**

- Yeasts in Food and Beverages. The Yeast Handbook. 2006, vol. 2
- Yeast technology. The Yeasts. 1993, vol. 5
- **G. ANNEMULLER** ; **H.J. MANGER** ; **P. LIETZ**. The yeast in the Brewery. 2018
- **RUT M.** Determination of nucleic acids on yeast and yeast related products.. *Kvasny Prumysl*, 1973, vol. 19, 131-133